# EUROPÄISCHE PATENTSCHRIFT

(11) **EP 0 678 010 B1**
(45) Veröffentlichungstag und Bekanntmachung des Hinweises auf die Patenterteilung: **15.01.1997**
(21) Anmeldenummer: 92910453.7
(22) Anmeldetag: 22.05.1992
(51) Int. Cl.: A61F 2/16

(54) **INTRAOKULARE KUNSTLINSE UND VERFAHREN ZU DEREN HERSTELLUNG**
INTRAOCULAR ARTIFICIAL LENS AND PROCESS FOR PRODUCING THE SAME
LENTILLE ARTIFICIELLE INTRA-OCULAIRE ET SON PROCEDE DE PRODUCTION

(30) Priorität: 23.05.1991 DE 4116869
(43) Veröffentlichungstag der Anmeldung: 25.10.1995
(73) Patentinhaber: BINDER, Helmut, 63179 Obertshausen (DE)
(72) Erfinder: BINDER, Helmut, 63179 Obertshausen (DE)
(74) Vertreter: Petra, Elke, Dipl.-Ing.
(86) Internationale Anmeldenummer: DE9200418
(87) Internationale Veröffentlichungsnummer: WO9220301

(56) Entgegenhaltungen:
- EP-A- 0 278 724
- WO-A-86/00520
- SU-A- 858 819
- SU-A- 1 634 271
- US-A- 3 975 779
- US-A- 4 242 760
- US-A- 4 504 981
- US-A- 4 662 882

## Beschreibung

Die Erfindung bezieht sich auf eine intraokulare Kunstlinse zum Ersatz der Augenlinse (Hinterkammer-Linse), wie sie beispielsweise bei Star-Operationen Verwendung findet.

Die bislang üblichen Methoden zur Implantation einer Kunstlinse genau an die Stelle der natürlichen Augenlinse, also in die sogenannte Hinterkammer, sind alle recht schwierig in der Durchführung, denn es gilt, die Kunstlinse sicher an dieser Stelle zu fixieren, ohne dabei Strukturen des Auges zu schädigen und gleichzeitig sowohl den zeitlichen, als auch den operativen Aufwand für den Eingriff möglichst gering zu halten.

Es sind verschiedene Implantations-Methoden unter Verwendung verschiedener Kunstlinsenarten bekannt, wie z.B. aus Fechner "Intraokularlinsen, Grundlagen und Operationslehre", 2. Auflage, Ferd. Enke Verlag, Stuttgart 1984, insb. S. 128 ff, hervorgeht.

So wird z.B. bei der sogenannten "extracapsularen Cataract-Extraktion", d.h. bei Erhalt der hinteren Linsenkapsel, die Implantation einer Kunstlinse vorgenommen, deren Haptiken (Befestigungsbügel) entweder im Kapselsack oder im Hinterkammergraben (Sulcus ciliaris) liegen.

Bei nicht erhaltener Linsenkapsel (sie reißt bei Entnahme der geschädigten Linse häufig ein) muß eine zusätzliche Abstützmöglichkeit verwendet werden. Beispielsweise kann eine Fixation der Kunstlinse durch Annähen der Haptiken an Augenstrukturen, z.B. am Ziliarkörper, vorgenommen werden. Hierdurch werden jedoch die Augenstrukturen geschädigt und es kann zu Blutungen im Augeninnern kommen. Zudem ist das Annähen schwierig und aufwendig und erfordert einen hohen zeitlichen Aufwand.

Auch besteht die Möglichkeit, die Fixierung durch Schaffung einer künstlichen Membran (Einziehen von Fäden) vorzunehmen, die verhindert, daß die Linse nach hinten in Richtung Glaskörper ausweichen kann. Diese Methode hat im wesentlichen die gleichen Nachteile, wie vorhergehend beschrieben.

Es ist daher bei jeder Operation als Ersatz vorgesehen, eine Kunstlinse möglichst an die originale Stelle der natürlichen Linse zu implantieren, die entweder auf der Iris, als sogenannter Iris-Clip, oder sogar davor liegt, als sogenannte Vorderkammer-Linse (EP-A1-03 46 245). Diese Vorderkammer-Linsen stellen eine ständige Gefahr für Strukturen des vorderen Augenabschnittes wie Hornhaut, Kammerwinkel und Iris dar. Auch durch den Iris-Clip wird die Funktion der Iris, insbesondere der Pupille, erheblich beeinträchtigt. Durch permanentes Reiben werden zwischen Linsen- und Irisoberfläche oft schwere Reizzustände provoziert.

Auch ist bekannt, die Abstützung der Kunstlinse durch Haptiken sowohl in der Vorderkammer als auch in der Hinterkammer vorzunehmen. So zeigen die US-PS 43 66 582 und US-PS 43 16 291 jeweils eine Linse mit Abstützung über zwei Haptiken in der Vorderkammer (geführt durch die Pupille und am äußeren Umfang der Iris anliegend) und über zwei weitere Haptiken in der Hinterkammer. In der US-PS 43 16 291 ist zusätzlich ein die Iris von vorn nach hinten durchdringender Dorn vorgesehen.

Aus der DE-PS 31 40 465 ist eine Linse bekannt, mit einem Stützelement für die hintere Augenkammer sowie einem Bügel, der durch einen Einschnitt in der Iris in die Vorderkammer geführt ist und dort ringförmig konzentrisch die Iris umgibt und nach Art eines Clips federnd gegen das Stützelement in der Hinterkammer drückt.

In der US-PS 42 42 760 ist eine Linse beschrieben, die ebenfalls in der Hinterkammer abgestützt ist. Eine der Haptiken reicht durch die Irisöffnung/Pupille, verläuft ein Stück radial vor derselben und tritt dann durch einen peripheren Einschnitt wieder in die Hinterkammer, wo dann das bogenför mige Endstück am Sulcus anliegt.

Des weiteren ist aus der US-A-4 504 981, und da insbesondere aus den Figuren 9 und 12, eine Kunstlinse bekannt, die zwei gegenläufige, spiegelsymmetrische Haptiken aufweist, mit zwei erkennbar unterschiedlichen Abschnitten und zwar einem von der Linsenkörperkante leicht konkav gebogenen Hinterkammer-Abschnitt und einen gegenläufigen, mit dem Vorderkammer-Winkel parallelen Vorderkammer-Abschnitt. Über einen Endbereich des Hinterkammer-Abschnitts treten diese Haptiken durch eine relativ periphere Iridektomie aus der Hinterkammer in die Vorderkammer, wo sich der Vorderkammer-Abschnitt auf der Iris aufliegend, abstützt. Somit ist diese bekannte Linser über die Vorderkammer-Abschnitte der Haptiken in der Iris, auf dieser sich gleichzeitig abstützend eingehängt, und eine Verschiebung bzw. ein Sich-Herausdrehen aus dieser Aufhängung der Linse bzw. deren Haptiken wird dadurch verhindert, daß diese gegenläufig ausgebildet sind. Eine besondere Elastizität müssen diese Haptiken nicht aufweisen, da sie sich in keinem der Augenkammerwinkel abstützen, sondern nur in der Iris eingehängt sind und durch ihre Gegenläufigkeit über die Irisdurchtritte festgeklemmt sind. Durch diese gegenläufige Ausführung der Haptiken ist jedoch deren Einbringung und Fixierung sehr komplex und können auch durch den Klemmeffekt in der Iris deren Bewegung behindern bzw. zu Reizungen führen. Eine radiale Positionsfixierung der Linsen wird nur durch die Anordnung der Iridektomien und Einhängung über diese der Haptiken vorgenommen. Eine elastische, radiale Abstützung, z.B. im sulcus ciliaris, durch welche eine genau zentrische Anordnung der Linse, unabhängig von der Realisierung der Iridektomien während der Operation, ist nicht sichergestellt.

Schließlich ist aus der US-PS 44 04 694 eine Linse bekannt, die ein in der Linsenkapsel gelagertes Haptikelement und ein die Iris im peripheren Bereich durchdringendes, mit seinem Endteil im Vorderkammerwinkel gelagertes zweites Haptikelement, aufweist.

Aufgabe der Erfindung ist es, eine intraokulare Kunstlinse anzugeben, die eine leichte, relativ schnelle Implantierbar keit, mit Anordnung des Linsenkörpers (Optik) in der Hinter kammer an der Stelle der natürlichen Linse, eine universelle Anwendbarkeit, eine sichere Fixation und eine möglichst geringe Gefährdung von Strukturen des Auges ermöglicht. Desweiteren soll ein wirtschaftliches Verfahren zur Herstellung der Kunstlinsen bereitgestellt werden.

Diese Aufgabe wird erfindungsgemäß durch eine intraokulare Kunstlinse mit den Merkmalen des Anspruchs 1 gelöst. Weitere vorteilhafte Ausgestaltungen sind in den Unteransprüchen beschrieben.

Der Kern der Erfindung ist im wesentlichen darin zu sehen, daß mindestens eine Haptik vorgesehen ist, die an dem in der Augen-Hinterkammer angeordneten Linsenkörper so befestigt ist, daß der Linsenkörper sowohl im Hinterkammer-Winkel (Sulcus ciliaris) als auch in der Vorderkammer abgestützt ist. Die Haptik weist dabei vom Linsenkörper ausgehend in Aufeinderfolge einen Hinterkammer-Abschnitt mit Hinterkammer-Abstütz-Segment, einen durch eine relativ periphere Iridektomie verlaufenden, aus der Linsenkörperebene herausgebogenen Durchtritts-Abschnitt und einen parallel zur Linsenkörperebene verlaufenden Vorderkammer-Abschnitt auf.
Hierdurch wird der Vorteil erreicht, daß einerseits die Optik (Linsenkörper) in der Hinterkammer, in der Position der entfernten natürlichen Linse liegt und andererseits die Fixierung der Linse über die mindestens eine Haptik in der Vorderkammer, von vorne sichtbar und gleichzeitig nicht nach hinten verrutschbar, also positionssicher, vorgenommen wird. Dabei kann die Abstützung durch die Haptik am peripheren Rand der Iris oder weiter radial auswärts, im Vorderkammerwinkel, vorgesehen werden.

In Weiterbildung des Erfindungsgedankens kann der Durchtrittsabschnitt im wesentlichen senkrecht oder in einem Winkel zur Iris durch zur Iridektomie verlaufen. Dabei kann der schräge bez. winklig angeordnete Durchtritts-Abschnitt im wesentlichen in einer senkrechten Durchmesserebene zum Linsenkörper bez. zur Iris verlaufen. Dabei ist selbstverständlich dessen Verlauf von dem Hinterkammer-Abschnitt zur Vorderkammer bez. zum Vorderkammer-Abschnitt radial-schräg nach oben bez. in Richtung auf die Vorderkammer. Hierzu ist von Vorteil, wenn der im wesentlichen bogenförmige Hinterkammerabschnitt über einen Einwärtsbogen mit dem Durchtrittsabschnitt verbunden ist. Somit besteht der große Vorteil, daß bei der Fertigung der erfindungsgemäßen Kunstlinse die Haptiken insgesamt in der Linsenebene aus vollem Material herausgearbeitet werden können, wonach lediglich der Durchtrittsabschnitt in die gewünschte Neigung hochgeformt werden muß. Dabei kann das Hochformen bez. Hochklappen des Durchtritt-Abschnitts bis in eine winklige oder in eine senkrechte Position vorgenommen werden.

Der schräg bez. winklig durch die Iridektomie hindurchgeführte Durchtritts-Abschnitt kann auch in einer im wesentlichen zur Iris tangential verlaufenden Ebene angeordnet sein. Dabei kann der Durchtritts-Abschnitt aus dem normalen Bogenverlauf des Hinterkammer-Abschnitts bis in die gewünschte Winkelposition herausgeformt sein.

Von besonderem Vorteil ist, wenn zwei oder mehr Haptiken an einem Linsenkörper angeordnet sind und wenn diese zueinander im wesentlichen identisch ausgebildet sind. Dadurch wird eine sehr sichere Positionierung sichergestellt. Sehr vorteilhaft wirkt sich aus, wenn die Abstützung durch die Haptiken jeweils gleichzeitig sowohl in der Vorderkammer als auch in der Hinterkammer vorgenommen wird. Dadurch wird eine sichere Fixation senkrecht zur Linsenebene optimal sichergestellt. Dies wird erreicht, wenn die Haptiken zusätzlich zu dem Abstützsegment in der Vorderkammer auch ein Abstütz-Segment in der Hinterkammer besitzten, über welche eine Abstützung im Hinterkammer-Winkel (Sulcus ciliaris) stattfindet.

Durch die erfindungsgemäße Zwei-Kammern-Abstützung der Haptiken besteht sogar die Möglichkeit, wenn durch die Abstützung zugleich ein Festklemmen der Iris zwischen den Abstütz--Segmenten stattfindet, an einem Linsenkörper (Optik) nur eine einzige Haptik vorzusehen. Dadurch spielt die erfindungsgemäße Klemm-Haptik die Rolle eines Clips, nur daß die Linse, nicht wie bei einem herkömmlichen Iris-Clip, in gleicher Ebene mit der Iris liegt, sondern in der natürlichen Linsenebene. Zudem wird die Iris nicht in ihrem zentralen Bereich, der für ihre Funktion von größter Bedeutung ist, sondern an der nicht so funktionswichtigen Peripherie festgeklemmt (keine Kontraktionsbehinderung der Pupille).

Eine optimale Positionierung/Fixation wird jedoch erzielt, wenn diese über mindestens zwei Haptiken vorgenommen wird. Eine besonders schonende Fixation wird dabei erreicht, wenn die Haptiken z.B. in Form einer archimedischen Spirale aus dem Linsenkörper heraustreten und die Abstützung über Segmente dieser Spirale geschieht. Hier ist wichtig, daß die jeweiligen Abstützsegment-Enden nach innen zumindest leicht zurückgesetzt sind, wodurch eine schonende Abstützung auch im Falle eines relativ kurzen Verlaufs möglich ist.

Gemäß einer anderen Weiterbildung des Erfindungsgedankens kann die Abstützung der Haptik über die Vorderkammer-Abschnitte an unterschiedlichen Zonen der Iris stattfinden.

So kann beispielsweise die Abstützung über die Vorderkammer-Abschnitte am peripheren Rand der Iris erfolgen, wobei diese im wesentlichen in Höhe der Iridektomie oder einem noch wei ter außen liegenden Irisabschnitt oder im Vorderkammer-Winkel erfolgen kann. Dies ist abhängig von der Ausbildung und vom Verlauf des Vorderkammer-Abschnitts. Eine besonders schonende, praktisch punktuelle Abstützung in der Vorderkam mer kann erreicht werden, wenn der Vorderkammer-Abschnitt in Form einer Schlaufe relativ weit an dem Durchtrittsab schnitt vorbei zurückgebogen ist, wobei der an den Durch tritts-Abschnitt anschließende Abschnitt entweder im Spiral verlauf oder gegenläufig zu diesem ausgebildet sein kann. Von Vorteil ist, wenn der rückführende Abstütz-Arm noch zusätzlich so geschweift ist, daß der Abbiegebogen/ Bogen-Abschnitt und das Abstütz-Ende jeweils in Abstützflächen richtung herausgebogen bzw. -geführt sind, jeweils eine nur sehr kurze, praktisch punktuelle Abstützung erlaubend. Erfindungsgemäß ist die Schleifen-Führung derart gewählt, daß das Rückführteil entweder peripher (außerhalb) oder zentral (innerhalb) in bezug auf den Durchtritts-Abschnitt verläuft. Bei innerem Verlauf des Schlaufenrückführarmes ist eine Zweipunktabstützung mit größter Sicherheit gewährleistet, auch wenn der Durchtrittssektor sehr nahe an der Peripherie verläuft. Eine flächige, entsprechend abgerundete Ausweitung des Abstütz-Endes, vorzugsweise mit einer darin vorgesehenen Handhabungsöffnung, wirkt sich sehr vorteilhaft aus. Die Enden können jedoch auch zu einem im wesentlichen geschlossenen Ring oder einer Öse zurückgebogen sein.

Im Rahmen der Erfindung sind die unterschiedlichsten Ausbildungen des Vorderkammer-Abschnitts möglich. So kann z.B. nur ein einziger, relativ kurzer Abstütz-Arm mit einem gegensinnig ausladenden, kürzeren Einhak-Arm vorgesehen sein. Möglich ist auch die Ausbildung mit zwei im wesentlichen im Verhältnis zum Durchtritts-Abschnitt symmetrischen Armen, die zum Hindurchführen durch die Iridektomie zusammenklappbar oder zusammenfaltbar ausgebildet sind. Der Vorderkammerabschnitt kann des weiteren in Form eines zum Hinterkammerabschnitt gegenläufigen Arm ausgebildet sein, wobei dieser Arm nach dem Austritt aus der Iritektomie einen im wesentlichen parallelen Verlauf zum Hinterkammer-Abschnitt oder einen tangentialen Verlauf aufweisen kann. Um eine sichere Einhängung zu gewährleisten, kann dabei am Anfang des Einhängearmes mindestens ein zu dem Einhängearm im wesentlichen senkrecht und somit im wesentlichen zum Linsenkörper bez. zur Iris radial verlaufender Querarm vorgesehen sein. Durch das Vorsehen von zwei identischen, radial gegenläufigen Querarmen ist eine besondere sichers Fixierung bez. Einhängung des Vorderkammer-Abschnitts sichergestellt. Selbstverständlich kann der Einhängearm auch im wesentlichen im weiteren Spiralverlauf des Hinterkammer-Abschnitts, also gleichläufig zum Hinterkammer-Abschnitt, ausgerichtet bzw. geführt sein, wobei ein Herausrutschen des Vorderkammer-Abschnitts durch die Iridektomie in die Hinterkammer, durch die Querarme sicher verhindert wird. Diese Ausführungsform läßt sich auch relativ wirtschaftlich herstellen, da jeweils eine Haptik mit entsprechend großer Länge und mit zwei kleinen Querarmen ausgeführt werden muß. Anschließend wird lediglich der Durchtritts-Abschnitt so herausgeformt, daß die Parallelausrichtung des Einhängearms beibehalten wird. Hierdurch wird das Heben nzw. Anordnen des Vorderkammer-Abschnitts in eine höhere Ebene, d. h. in der Iris-Ober flächenebene, realisiert wird.

Der Vorderkammer-Abschnitt kann jedoch auch lediglich aus zwei im Verhältnis zur Iridektomie bzw. zum Durchtritts-Abschnitt gegenläufigen bzw. symmetrischen Einhängearmen bestehen, wodurch eine Vorderkammer-Aufhängung entsteht. Dabei kann diese aus zwei Einhängearmen bestehende Vorderkammer-Aufhängung an jeder beliebigen Ausführungsform des Durchtritts-Abschnitts angeordnet sein. So kann in einer Ausführungsform der Hinterkammer-Abschnitt im wesentlichen spiral- oder bogenförmig bis im wesentlichen unter die Iridektomie geführt sein, wonach der Durchtritts-Abschnitt beispielsweise senkrecht zur Iris ausgerichtet ist. Dieser kann jedoch auch aus dem Verlauf des Hinterkammer-Abschnitts heraus schräg zur Iris ausgerichtet sein.

In einer weiteren Ausführungsform kann die Vorderkammer-Aufhängung an einem schräg-radialen Durchtritts-Abschnitt angebracht sein, der über einen Einwärtsbogen mit dem Hinterkammer-Abschnitt in Verbindung steht. Diese Ausführungsform ist ebenfalls eine herstellungsgünstige Variante, da hier die Möglichkeit besteht, die Haptiken in der Linsenebene herkömmlich durch Drehen und Fräsen aus einem Materialstück herauszuarbeiten. Danach wird der Durchtritts-Abschnitt aus der Linsenebene schräg in einer Radialebene so herausgebogen, daß die doppelarmige Aufhängung parallel zur Ausgangsposition verschwenkt wird. Diese Ausführungsform kann auch in herkömmlicher, nur spanabhebender Weise optimal hergestellt weden, wenn die Schräge des Durchtritts-Abschnitts so groß ist, daß Aufhängung und Durchtritts-Abschnitt nicht in der gleichen Tangential-Vertikal-Ebene liegen und somit praktisch kein Gratmaterial unterhalb der Aufhängungsarme nachträglich in einem separaten Arbeitsgang entfernt werden muß.

Die Vorderkammer-Aufhängung kann auch so ausgebildet sein, daß die beiden Einhängearme im wesentlichen radial zur Iris bzw. zum Linsenkörper ausgerichtet sind. Dabei ist jedoch sinnvoll, diese Arme relativ kurz zu halten, d.h. der äußere, periphere Arm muß kürzer sein als der Abstand der Iridektomie zum Vorderkammer-Winkel. Um eine sichere Aufhängung und gleichzeitig ein irritierendes Anstoßen am Vorderkammer-Winkel zu vermeiden, kann zumindest der nach außen weisende Einhängearm bogenförmig zurück oder nach vorne, im Vergleich zum Verlauf des Hinterkammer-Abschnitts, gebogen sein. Fertigungsmäßig besteht hier ebenfalls eine vorteilhafte Ausführungsmöglichkeit (bei spanabhebender Fertigung mit anschließendem thermischem Verformen), da der Durchtritts-Abschnitt aus dem Hinterkammer-Abschnitt entsprechend winklig herausgebogen werden kann. Bei Ausführung der Einhängearme mit bogenförmigen Enden ist sinnvoll, diese über entsprechende Gegenbiegungen erneut in eine im wesentlichen parallele Ebene zur Linsen-bzw. Irisebene zurückzuführen.

Erfindungsgemäß beginnt der Vorderkammer-Abschnitt vorzugsweise mit einem Radialteil, der im wesentlichen radial und zugleich parallel zur Iris verläuft. D.H., die Haptik verläuft mit ihrem Durchtrittsabschnitt senkrecht oder leicht schräg zur Iris durch die Iridektomie und über dieser, nur relativ kurz, gleichzeitig im wesentlichen parallel zur Iris, im wesentlichen radial nach außen oder radial nach innen (Radialteil), um anschließend in die vorbeschriebenen, nachfolgenden Abstütz-, Einhänge- bzw. aufhängearme, bzw. -Teile des Vorderkammer-Abschnitts überzugehen.

Der Radialteil kann dabei eine nur relativ kleine Längs- bzw. Radialerstreckung aufweisen. Es genügt, wenn durch den Radialteil der nächstfolgende Abschnitt so beginnt, daß er nicht in der gleichen Tangentialebene mit dem Durchtritts-Abschnitt liegt. Es reicht somit aus, wenn der Radialteil mindestens eine Länge aufweist, die gleich dem Durchmesser des Durchtritss-Abschnitts ist. Dies ist z.B. der Fall, wenn der nächste Teil des Vorderkammer-Abschnitts, z.B. die zweiarmige Aufhängung nicht T-förmig obenauf auf dem Durchtritts-Abschnitt aufsitzt, sondern im wesnetlichen tangential am Außenumfang/Mantelfläche seines äußeren Endes. Durch diese Ausbildung der Haptik, insbesondere ihres Vorderkammer-Abschnitts, bleibt bei herkömmlicher Herstellungsweise der Kunstlinse nur durch Drehen und anschließendes Fräsen, kein nachträglich zu entfernendes Material/Grat zwischen Durchtritts- und Vorderkammerabschnitt stehen. Eine nachträgliche Gratentfernung ist wegen der sehr geringen Abmessungen der Kunstlinse sehr zeit- und kostenintensiv und mit großem Bruchrisiko verbunden.

Wie bereits vorerwähnt, kann der Radialteil nach innen oder nach außen verlaufen, in Abhängigkeit von der Form der weiteren Teile des Vorderkammer-Abschnitts und/oder je nach beabsichtigtem Radialabstand der Iridektomie vom Irisrand. Es ist ersichtlich, daß aus Fertigungsgesichtspunkten der radial nach außen führende Verlauf des Radialteils vorteilhafter ist, insbesondere, wenn der Hinterkammer-abschnitt in einem Einwärtsbogen endet. Ein einwärts geführter Radialteil kann jedoch dann von Vorteil sein, wenn kein nauch außen offener Einwärtsbogen vorhanden ist, sondern der Hinterkammerabschnitt z. B. lediglich seine leichte Spiral-Einwärtskrümmung aufweist oder diese Krümmung am Abschnitts-Ende leicht einwärts verstärkt ist.

Die Form der Haptiken ist folglich erfindungsgemäß so gewählt, daß sie den in der Hinterkammer befindlichen Linsenkörper sowohl im Sulcus ciliaris als auch gleichzeitig in der Vorderkammer abstützen. Dabei führt die Verlängerung der im Sulcus liegenden Bügel durch periphere Öffnungen in der Iris (Iridektomien) in die Vorderkammer, so daß die speziell geformten Enden der Haptiken z. B. im Vorderkammer-Winkel zu liegen kommen oder oberhalb der Iris aufliegen. Die Vorteile dieser Konstruktion sind u.a. :
1. der Linsenkörper befindet sich genau an der Stelle der natürlichen Linse;
2. die Abstützung erfolgt sehr schonend im Sulcus und eventuell punktuell im Vorderkammer-Winkel, oder eingehängt auf sehr kurzem Abschnitten der Iris;
3. die Iris wird praktisch nicht in ihrer Funktion beeinträchtigt, da es nur punktweise oder in nur sehr geringen Abschnitten zu Berührungen zwischen Haptiken und Iris kommt und die Funktion (Erweiterung, Verengung der Pupille) voll gewährleistet ist;
4. die Linse ist für alle Fälle, die das Implantieren einer Kunstlinse notwendig machen, anwendbar, da sie völlig unabhängig vom Vorhandensein oder Nicht-Vorhandensein einer haltgebenden Membran (hinterer Teil der Linsenkapsel) ist;
5. die Linse ist leicht implantierbar:
   - es ist keine Hilfsoperation notwendig,
   - das Einführen in die Hinterkammer ist ähnlich wie bei herkömmlichen Hinterkammerlinsen,
   - das Anbringen von Iridektomien ist Standard bei nahezu allen Linsenimplantationen,
   - sehr unbefriedigend bei herkömmlichen Hinterkammerlinsen ist die Ungewißheit über die Lage der Haltebügel, da diese nach der Implantation nicht mehr sichtbar sind. Schlecht sitzende Bügel können zu erheblichen Problemen führen (z.B. sogenannte "gefangene Iris"). Bei der erfindungsgemäßen Linse sind die Haltebügel jedoch gut sichtbar und ihre Stellung intraoperativ gut justierbar;
6. die Linse ist universell anwendbar in allen Fällen:
   - bei "extracapsulärer Cataract-Extraction" (ECCE) als sogenannte "stand by"-Linse,
   - bei allen "intracapsulären Cataract-Extractionen" (ICCE) (komplette Entfernung des Kapselsackes),
   - bei allen Sekundäroperationen,
   - zum Schutz der Iris bei Operationen an den hinteren Augenabschnitten mit Silikonersatz des Glaskörpers,
   - als Ersatz der Vorderkammerlinsen und der sogenannten Irisclip-Linsen;
7. möglichst geringe Gefährdung von Strukturen des Auges, z.B. durch Reibung an empfindlichen Oberflächen, durch Verlegen des Kammerwasserflusses, u.a.m.;
8. die Linse ist äußerst gut fixiert:
   - der in der Vorderkammer liegende Teil der Haptiken verhindert eine Bewegung nach hinten in Richtung Glaskörper und gibt ebenfalls eine Umfangs-/Dreh-Stabilität; folglich kein "Verlieren" im Glaskörper möglich;
   - zudem ist durch die spezielle Schleifen-, Haken- oder Bügelform des in der Vorderkammer liegenden Teils der Haptiken ein Weggleiten durch die Iridektomien nicht möglich.

Erfindungsgemäß ist die Kunstlinse in einer vorzugsweisen Ausführung mit der mindestens einen Haptik einstückig ausgebildet. D.h., die intraokulare Kunstlinse wird aus einem Materialstück herausgearbeitet.

Der Linsenkörper ist in weiterer Ausführungsweise mit der mindestens einen Haptik mehrstückig ausgebildet, wobei die mindest eine Haptik am Linsenkörper in an sich bekannter Weise, beispielsweise durch direktes Ankleben oder Einführen des Haptikendes in eine entsprechende Umfangsbohrung und anschließendes Verkleben, befestigt ist.

Insgesamt kann gesagt werden, daß mit der erfindungsgemäßen Kunstlinse die Operation am Auge wesentlich vereinfacht und verkürzt wird. Zudem wird ein besseres Ergebnis erzielt und vor allem die Langzeitprognose wesentlich verbessert. Es ist sogar denkbar, daß von der heute als am besten befundenen Methode (ECCE) abgegangen werden kann, da der Erhalt des hinteren Kapselteiles (der verbleibende Kapselteil kann fibrosieren und einen späteren, zweiten Eingriff -Kapsulotomie notwendig machen) nicht mehr notwendig ist. Diese Operation mißlingt häufig, da die Kapsel einreißt. Außerdem bleiben oft Linsenreste übrig, die später Probleme bereiten können. Dies alles ist bei der erfindungsgemäßen Linse nicht der Fall, da die natürliche Linse einfach komplett mit der Kapsel entfernt werden kann. Die erfindungsgemäße Kunstlinse kann somit ohne Einschränkung praktisch bei allen Linsenersatz-Operationen verwendet werden.

Die Aufgabe wird desweiteren durch ein Verfahren zur Herstellung der vorbeschiebenen intraokularen Kunstlinse mit den Merkmalen des Anspruchs 35 gelöst.

Demgemäß wird der Linsenkörper zusammen mit den Haptiken durch Drehen und nachfolgendes Fräsen in ersten Schritten so hergestellt, daß die Haptiken mit allen Ihren Abschnitten (Hinterkammer-, Durchtritts- und Vorderkammer-Abschnitt) im wesentlichen in der Linsenkörperebene liegen. Danach wird in mindestens einem Folge-Schritt abschnittsweise zumindest der Durchtritts-Abschnitt aus der Linsenebene in eine senkrechte oder winklige Position herausgeformt bzw. -geschwenkt. Dies kann je nach verwendetem Material z. B. thermisch erfolgen. Je nach Form des Vorderkammer-Abschnitts kann zusätzlich auch dieser anschließend verformt, bzw. in eine zur Linsenebene parallele Ausrichtung wieder zurückgeführt werden.

Hier wird erneut darauf hingewiesen, daß durch Anordnung eines Radialteils am Anfang des Vorderkammer-Abschnitts, es möglich ist, die Kunstlinse in einer sehr vorteilhaften, herkömmlichen Weise herzustellen, nämlich nur durch Drehen und Fräsen, ohne nachträgliches Gratentfernen.

Nachfolgend wird die Erfindung anhand von Ausführungsbeispielen unter Bezug auf die Zeichnung näher erläutert.

Es zeigt:
- Fig. 1:: eine Draufsicht auf eine Kunstlinse mit zwei Haptiken mit peripher verlaufendem Rückführarm des Vorderkammer-Abschnittes,
- Fig. 2:: eine Draufsicht wie in Fig. 1, mit zentral verlaufendem Abschnitt, wobei jede der zwei Haptiken eine andere Ausführungsform aufweist,
- Fig. 3:: eine Draufsicht wie in Fig. 1, mit dem Vorderkammer-Abschnitt in weiterer Ausführung (mit einem Abstütz-Arm und Gegenhaken),
- Fig. 4:: eine Ansicht nach Pfeil IV aus Fig. 3,
- Fig. 5:: einen Schnitt nach den Linien V-V aus Fig.1, gleichzeitig einen teilweisen Schnitt durch einen Augapfel mit implantierter Kunstlinse darstellend,
- Fig. 6:: eine Draufsicht auf eine Kunstlinse in weiterer Ausführungsform, mit einem tangentialen Vorderkammer-Einhängearm und zwei Querarmen,
- Fig. 7:: eine Draufsicht auf eine weitere Ausführungsform der Kunstlinse, mit zwei tangentialen Vorderkammer-Einhängearmen und schrägem Durchtritts-Abschnitt,
- Fig. 8:: eine weitere Ausführungsform der Kunstlinse mit Vorderkammer-Eingängearmen wie in Fig. 8, mit senkrechtem Durchtritts-Abschnitt,
- Fig. 9:: einen Schnitt nach IX-IX durch die Kunstlinse aus Fig. 8,
- Fig.10:: eine perspektivische Ansicht der Kunstlinse aus Fig. 8, die Ausrichtung der Haptik-Abschnitte veranschaulichend,
- Fig.11:: eine Draufsicht auf eine weitere Ausführungsform der Kunstlinse, mit Vorderkammer-Aufhängung, radialschrägem Durchtritts-Abschnitt und Einwärtsbogen,
- Fig.12:: eine Seitenansicht der Kunstlinse nach Fig. 11,
- Fig.13:: einen Schnitt nach XII-XII in Fig. 11, und
- Fig.14:: eine perspektivische Ansicht der Kunstlinse nach Fig. 11, den Verlauf der Haptik-Abschnitte verdeutlichend.
- Fig.15:: eine Draufsicht auf eine Kunstlinse in einer anderen Ausführungsform, mit einem Radialteil am Vorderkammer-Abschnitt,
- Fig. 16:: einen Schnitt nach XVI-XVI in Fig. 15, und
- Fig. 17:: eine Perspektivansicht der Kunstlinse nach Fig. 15, die Haptik-Ausbildung verdeutlichend.

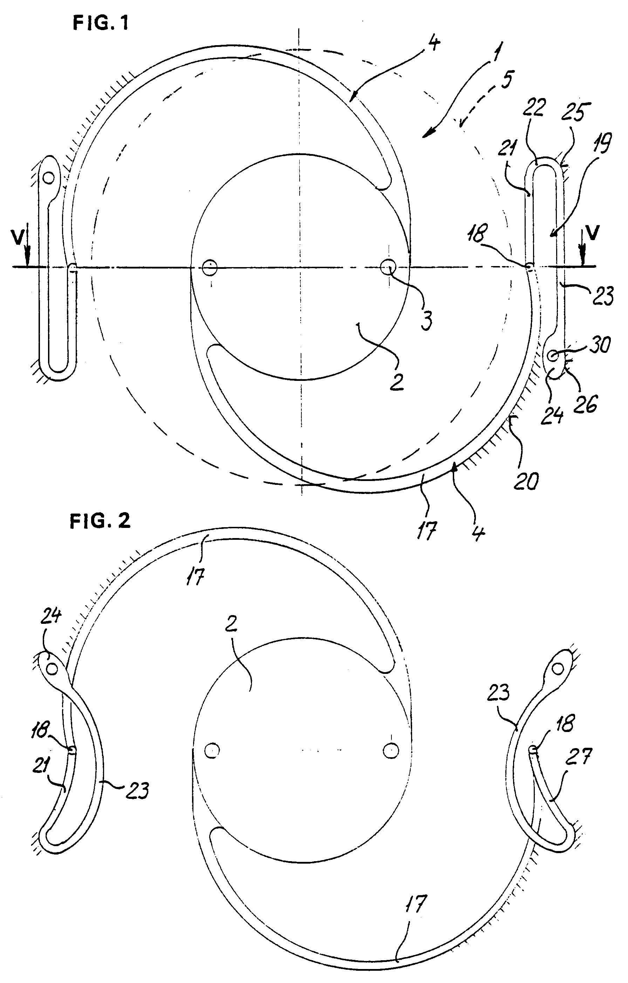

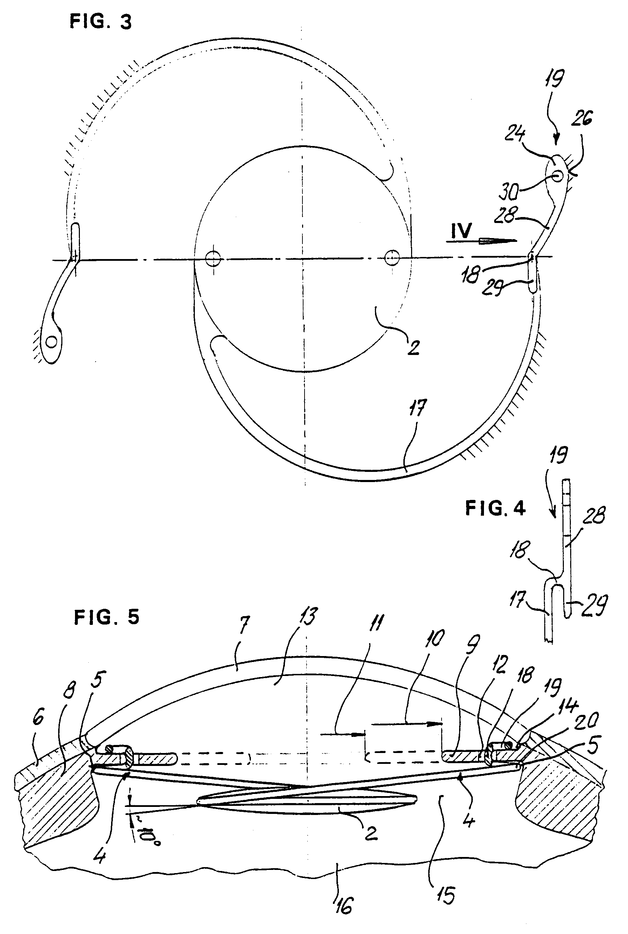

Aus Fig. 1 in Verbindung mit Fig. 3 wird ersichtlich, daß die erfindungsgemäße intraokulare Kunstlinse 1 zusammengesetzt ist aus einem diskusförmigen Linsenkörper 2, in dem mindestens zwei Handhabungsöffnungen 3 vorgesehen sind. In der Zeichnung nicht dargestellt ist die im Linsenkörper 2 in an sich bekannter Weise eingebrachte Optik der Linse.

Am äußeren Umfang des Linsenkörpers 2 sind jeweils rotationssymmetrisch zwei im wesentlichen spiralförmig verlaufende Haptiken (Befestigungs-Bügel) 4 angeformt. Die Haptiken 4 sind relativ dünn und elastisch ausgelegt. Sie können sich mühelos in Richtung Linsenkörper 2 zusammendrücken und in beispielsweise den mit unterbrochenen Linien eingezeichneten Sulcus ciliaris (Hinterkammer-Graben) 5 einlegen lassen.

Um den Aufbau der erfindungsgemäßen Kunstlinse und insbesondere deren besondere Befestigungsart im Auge genauer beschreiben zu können, soll vorab kurz auf den Aufbau des vorderen Teiles des Augapfels, wie in Fig. 5 im Schnitt dargestellt, näher eingegangen werden.

Der Augapfel wird durch die Sklera/Lederhaut 6 umgeben, die an ihrem vorderen Teil in die durchsichtige Hornhaut 7 übergeht. An der der Hornhaut benachbarten Innenseite der Sklera 6 befindet sich der im wesentlichen ringförmige Ziliarkörper 8, der in die Iris/Regenbogenhaut übergeht. Der Ziliarkörper 8 hat zur Iris hin eine ringförmige Einbuchtung, den Sulcus ciliaris/Hinterkammer-Winkel 5. Die Iris 9 kann sich bekannterweise ringförmig öffnen und schließen und dabei die Positionen "Pupille offen" 10 oder "Pupille zusammengezogen" 11 (strichliert eingezeichnet) einnehmen. Bei Augenoperationen werden fast immer, vorzugsweise am peripheren Rand der Iris 9, Iridektomien/Durchgangsöffnungen 12 zum Flüssigkeitsdurchtritt eingebracht.

Die Iris 9 teilt den Vorderabschnitt des Augapfels in eine Vorderkammer 13 und eine Hinterkammer 15. Die von vorne einsehbare Vorderkammer 13 bildet zwischen Iris 9 und Hornhaut 7 einen ringförmigen Vorderkammer-Winkel 14. Hinter der Iris 9, in Richtung auf den Glaskörper 16, befindet sich die Hinterkammer 15, die, wie bereits vorher erwähnt, zwischen Ziliarkörper 8 und Iris 9, den Sulcus ciliaris/Hinterkammer-Winkel 5 aufweist.

Aus Fig. 1 in Verbindung mit Fig. 5 wird ersichtlich, daß die Haptiken 4 aus der Sicht ihres Verlaufs in den beiden Augenkammern in verschiedene Abschnitte eingeteilt werden können. So kann von einem Hinterkammer-Abschnitt 17, der eine Neigung zur Linsenebene von ca. 10° aufweist, von einem Durchtritts-Abschnitt 18, der im wesentlichen senkrecht zur Linsenebene verläuft und schließlich von einem Vorderkammer-Abschnitt 19, mit im wesentlichen parallelen Verlauf zu dem Linsenkörper 2, gesprochen werden.

Der Hinterkammer-Abschnitt 17 ist auf seinem dem Durchtritts-Abschnitt 18 benachbart liegenden Ende vom normalen spiralförmigen Verlauf abweichend, etwas radial nach innen gedrückt, so daß, in im Hinterkammer-Winkel/Sulcus ciliaris 5 eingelegtem Zustand, sich der Hinterkammer-Abschnitt 17 nur über einen Teil seines Umfangs, dem Hinterkammer-Abstützsegment 20, wie in der Zeichnung mit kurzer Schraffur angedeutet, abstützt.

Der Vorderkammer-Abschnitt 19 kann erfindungsgemäß in unterschiedlicher Weise ausgebildet sein, wie aus Figuren 1-4 ersichtlich.
So zeigt Fig. 1 ein Ausführungsbeispiel und Figur 2 zwei unterschiedliche Ausführungsbeispiele von Vorderkammer-Abschnitten 19, bei denen jeweils der Abschnitt 19 einen im wesentlichen schlaufenförmigen Verlauf aufweist. Wesentlich dabei ist jeweils, daß der Vorderkammer-Abschnitt in seinem ersten Verlaufsteil, vom Durchtritts-Abschnitt 18 ausgehend, einen Anfangs-Arm 21 aufweist, der über einen Abstütz-Bogen 22 in einen zum Arm 21 rückläufigen Abstütz-Arm 23, mit einem Abstütz-Ende 24, übergeht. Der Abstütz-Arm 23 stützt sich im Vorderkammer-Winkel 14 über die sehr kurzen, mit Schraffurlinien angedeuteten Segmente, dem Abstützbogen-Segment 25 und dem Abstützende-Segment 26, praktisch punktuell ab.

In dem in Fig. 1 dargestellten Ausführungsbeispiel der Vorderkammer-Abschnitte 19 der Haptiken 4 ist der rückläufige Abstütz-Arm 23 peripher in bezug auf den Durchtritts--Abschnitt 18 angeordnet, während in den in Fig. 2 dargestellten Ausführungsbeispielen die Abstütz-Arme 23 jeweils zentral, d.h. nach innen verlaufend, geführt sind. Dabei ist der Anfangs-Arm 21 des in Fig. 2 dargestellten linken Vorderkammer-Abschnittes, ähnlich wie bei dem Ausführungsbeispiel in Fig. 1, im wesentlichen im Spiralverlauf der Haptik geformt, während die in Fig. 2 rechts dargestellte Ausführungsform einen Anfangs-Arm 27 aufweist, der in Spiralgegenlauf geformt ist.

In Figuren 3 und 4 ist eine weitere konstruktive Ausgestaltung des Vorderkammer-Abschnitts 19 dargestellt. Dieses vierte Ausführungsbeispiel weist keine schlaufenförmige Ausbildung auf, sondern lediglich einen Abstütz-Arm 28, der im wesentlichen der Endhälfte des in Fig. 1 dargestellten Abstütz-Armes 23 entspricht und in ein erweitertes Abstütz-Ende mit einem Abstütz-Segment 26 ausläuft. In im wesentlichen entgegengesetzter Richtung zum Abstütz-Arm 28 ist ein kurzer Hakenarm 29 angeformt, der ein Herausrutschen des Abstütz-Armes 28 durch die Iridektomie 12 verhindert. In den jeweiligen Abstützenden 24 der Abstütz-Arme 23 bzw. 28 können ebenfalls Handhabungsöffnungen 30 vorgesehen sein, die ein leichteres Einbringen des Vorderkammer-Abschnittes in die Abstützposition erlaubt.

Das Abstütz-Ende 24 kann, statt als Erweiterung mit Handhabungsöffnung 30, als Öhr oder in Form eines ringförmigen, im wesentlich zugezogenen Bogens, ausgebildet sein.

Die Einbringung/Fixation der erfindungsgemäßen intraokularen Kunstlinse ist sehr einfach. Vorhergehend sind an der Peripherie der Iris 9, je nach Anzahl der Haptiken 4 gleichmäßig verteilt zwei oder mehr Iridektomien 12 einzubringen. Dies ist Standard und wird bei fast jedem Eingriff am Auge durchgeführt. Nach Einführen der Kunstlinse 1 in die Hinterkammer 15, mit leicht zusammengedrückten Haptiken 4, werden die Abstütz-Enden 24 jeweils durch die Iridektomien 12 hindurchgesteckt und, beispielsweise bei dem in Fig. 1 dargestellten Ausführungsbeispiel, zuerst im Uhrzeigersinn gedreht, bis die Abstütz-Arme 23 bis zum Bogenabschnitt 22 herausstehen. Danach wird im Gegenuhrzeigersinn eine Drehung ausgeführt, bis die Anfangs-Arme 21 ganz in der Vorderkammer 14 erscheinen und die Durchtritts-Abschnitte 18 in die Iridektomien 12 eingezogen sind. Dies sind ganz leichte Dreh-Verschiebe-Bewegungen, die insbesondere durch die Handhabungsöffnungen 3 und 30 erleichtert werden.

Bei dem Ausführungsbeispiel nach Fig. 3 ist nach Einführen der Abstütz-Enden 24 durch die Iridektomie 12 lediglich ein Verschieben im Gegenuhrzeigersinn vorzunehmen, bis auf Anschlag der Iridektomie mit dem Durchtritts-Abschnitt 18, wonach die Iridektomie der sehr elastischen Iris 9 erweitert über den Hakenarm 29 gezogen wird, diesen freigebend, wonach sich die Iridektomie bzw. die Iris elastisch um den Durchtritts-Abschnitt 18 legt.

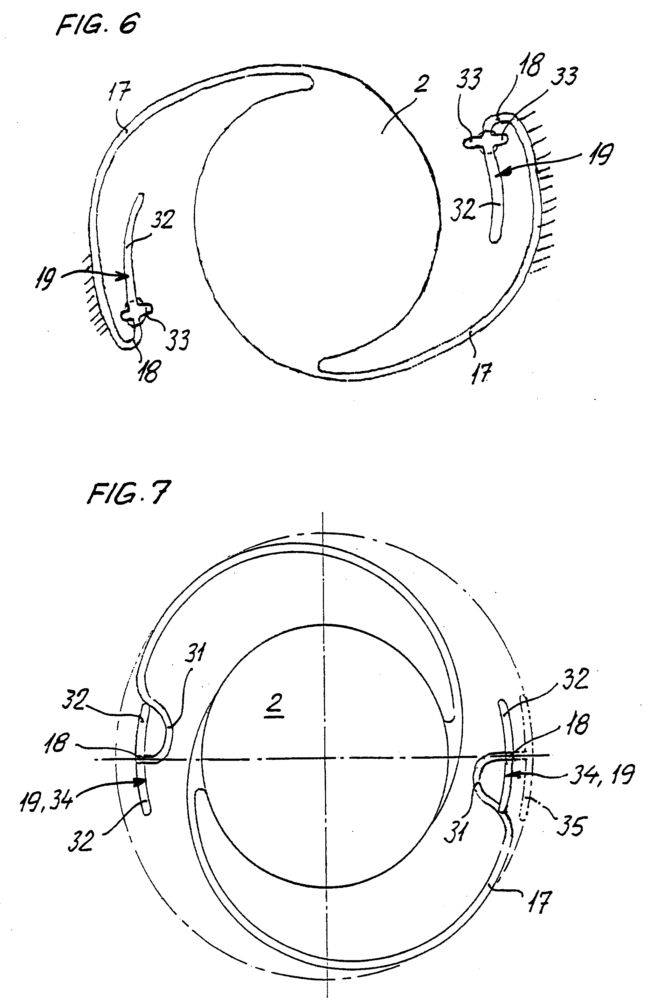

In dem in Fig. 6 dargestellten weiteren Ausführungsbeispiel besteht der Vorderkammer-Abschnitt 19 aus einem Einhängearm 32, der im wesentlichen gegenläufig zum Hinterkammer-Abschnitt 17 ausgerichtet ist. Dieser kann selbstverständlich auch gleichläufig ausgerichtet sein. An dem dem Durchtrittsabschnitt 18 benachbarten Ende weist der Einhängearm 32 zwei im wesentlichen radial ausgerichtete Querarme 33 auf, durch die ein Herausrutschen des Einhängearms 32 durch die Iridektomie 12 verhindert wird.

Bei dem in Fig. 7 dargestellten Ausführungsbeispiel der Kunstlinse besteht der Vorderkammer-Abschnitt 19 aus zwei gegenläufigen, zum Durchtritts-Abschnitt 18 symmetrischen Einhängearmen 32, die im wesentlichen parallel und auf Abstand zum Vorderkammer-Winkel angeordnet sind und eine Vorderkammer-Aufhängung 34 bilden. Der Durchtritts-Abschnitt ist bei dieser Ausführungsform senkrecht zur Iris ausgebildet. Dabei ist das dem Durchtritts-Abschnitt 18 benachbarte Ende des Hinterkammer-Abschnitts 17 als Einwärtsbogen 31 ausgebildet, dessen Bogenende im wesentliche radial nach außen weist.
Bei dem in Fig. 7 rechts dargestellten Haptikende ist die Vorderkammer-Aufhängung 34, 35 zusätzlich in Strich-Punkt-Linie eingezeichnet. Diese Darstellung zeigt den Vorderkammer-Abschnitt 19 bzw. die Aufhängung 34, 35 in einer Herstellungsphase, in der diese zusammen mit dem Durchtritts-Abschnitt 18 noch nicht aus der ersten Bearbeitungsebene (z. B. spanende Bearbeitung) in die planparallel versetzte Funktionsebene des Vorderkammer-Abschnitts mit senkrechter oder winkliger Position des Durchtritts-Abschnitts verformt worden sind.

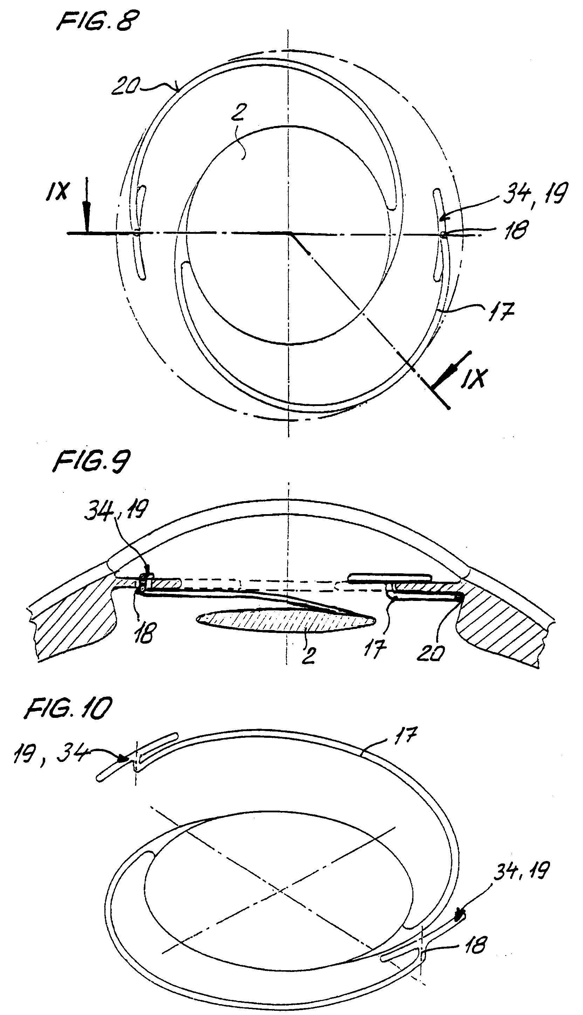

In dem in Figuren 8- 10 dargestellten Ausführungsbeispiel ist der Vorderkammer-Abschnitt 19 im wesentlichen identisch mit demjenigen aus Fig. 7. Hier ist jedoch der Durchtritts-Abschnitt 18 direkt aus dem Verlauf des Hinterkammer-Abschnittes herausgebogen (im wesentlichen tangential verschwenkt) und zwar im wesentlichen senkrecht zur Iris verlaufend. Dabei ist insbesondere in der Perspektivansicht aus Fig. 10 die Anordnung und Ausbildung der Haptik-Abschnitte gut erkennbar.

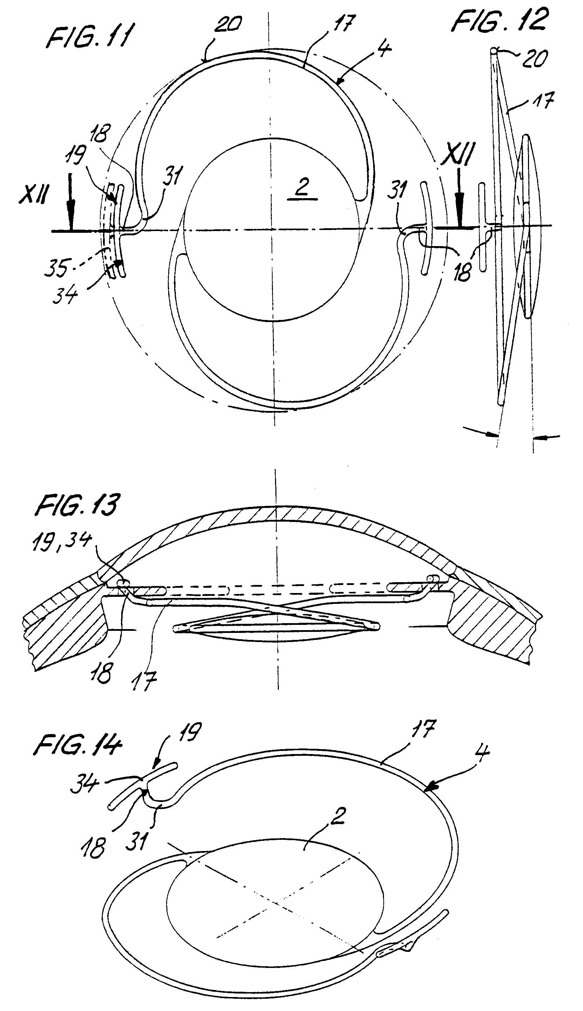

In dem in Figuren 11 - 14 dargestellten Ausführungsbeispiel handelt es sich um eine weitere Variante zu dem Ausführungsbeispiel nach Fig. 7. Während im Ausführungsbeispiel nach Fig. 7 der Durchtritts-Abschnitt 18 im wesentlichen senkrecht zur Iris verläuft, ist bei dem in Figuren 11-14 dargestellten Beispiel der Verlauf des Durchtritts-Abschnitts 18 im wesentlichen schräg- bzw. radial-winklig zur Iris. Da ist der Durchtritts-Abschnitt 18 praktisch direkt aus dem Einwärtsbogen 31 herausgebogen (radial verschwenkt), wie dies insbesondere in Figuren 11, 13 und 14 gut sichtbar ist. In Fig. 11 ist an der linken Haptik mit unterbrochener Linie die Position der Vorderkammer-Aufhängung 34 vor dem Herausbiegen aus der Linsenebene dargestellt. Es wird ersichtlich, daß eine relativ günstige Fertigung in der Linsenebene, mit anschließendem Herausbiegen des Durchtritts-Abschnitts 18, möglich ist.

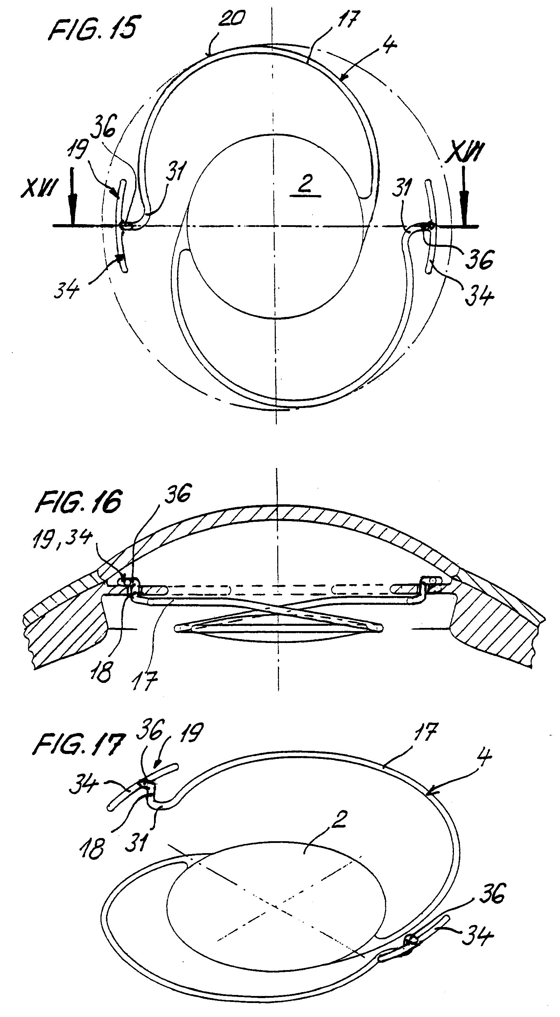

Aus Figuren 12 und 13 im Vergleich mit insbesondere Figuren 5 und 9 wird ersichtlich, daß auch die Formgebung des Hinterkammer-Abschnitts insbesondere in bezug auf seine Winkelausrichtung zur Iris unterschiedlich ausgelegt sein kann. So kann beispielsweise ein Hinterkammer-Abschnitt eine konstante Neigung aufweisen, wie aus Fig. 5 ersichtlich ist. Der Hinterkammer-Abschnitt kann jedoch auch in seinem ersten Abschnitt, im wesentlichen bis zum Abstützsegment 20 stärker geneigt sein, während der weitere, bis zum Durchtritts-Abschnitt führende Teil, im wesentlichen parallel zur Iris verläuft.

Bei der in Fig. 15 bis 17 dargestellten Ausführungsform handelt es sich um eine weitere Variante zu den Ausführungsbeispielen nach Fig. 7 sowie Fig. 11 bis 14. Es ist erkennbar, daß der Vorderkammer-Abschnitt 19, der hier eine doppelarmige Aufhängung 34 aufweist, noch zusätzlich zwischen der Aufhängung 34 und dem Durchtritts-Abschnitt 18 einen Radialteil 36 besitzt. Durch den Radialteil 36, der in gleicher Horizontal-Ebene mit der Aufhängung 34 und in im wesentlichen gleicher Radial-Vertikal-Ebene mit dem Durchtritts-Abschnitt 18 liegt, erfährt die Aufhängung 34 einen radialen Versatz nach außen. Es ist erkennbar, daß der Radialteil 36 auch nach innen verlaufen angeordnet sein kann, wodurch die Aufhängung eine Innenlage im Verhältnis zur Iridektomie 12 bzw. zum Durchtritts-Abschnitt erhält.

Der Radialteil 36 kann an sich bei jeder der in den vorbeschiebenen Figuren dargestellten Kustlinsen vorgesehen sein. Hinterkammer-Abschnitts-Ende, Durchtritts-Abschnitt und Vorderkammer-Abschnitt (mit Radialteil) müssen jeweils so ausgebildet bzw. zueinander angeordnet sein, daß keine; der Abschnittsteile in der gleichen Tangential-Vertikal-Ebene zu liegen kommen.

### Bezugszeichenliste

- 1.: Kunstlinse
- 2.: Linsenkörper
- 3.: Handhabungsöffnungen
- 4.: Haptiken
- 5.: Hinterkammer-Winkel/Sulcus ciliaris
- 6.: Lederhaut/Sklera
- 7.: Hornhaut/Cornea
- 8.: Ziliarkörper
- 9.: Regenbogenhaut/Iris
- 10.: Pupille offen
- 11.: Pupille zugezogen
- 12.: Iridektomie/Iris-Durchtrittsöffnung
- 13.: Vorderkammer
- 14.: Vorderkammer-Winkel
- 15.: Hinterkammer
- 16.: Glaskörper
- 17.: Hinterkammer-Abschnitt
- 18.: Durchtritts-Abschnitt
- 19.: Vorderkammer-Abschnitt
- 20.: Hinterkammer-Abstützsegment
- 21.: Anfangs-Arm
- 22.: Abstütz-Bogen
- 23.: Abstütz-Arm
- 24.: Abstütz-Ende
- 25.: Abstütz-Bogen-Segment
- 26.: Abstütz-Ende-Segment
- 27.: Anfangs-Arm, gegenläufig
- 28.: Abstütz-Arm
- 29.: Haken-Arm
- 30.: Handhabungsöffnung
- 31.: Einwärtsbogen
- 32.: Einhängearm
- 33.: Querarm
- 34.: Aufhängung (Vorderkammer-)
- 35.: Aufhängung (Vorbiegen)
- 36.: Radialteil

## Patentansprüche

1. Intraokulare Kunstlinse zum Ersatz der Augenlinse,
- mit einem in der Augen-Hinterkammer anzuordnenden Linsenkörper (2),
- und mit mindestens einer am Linsenkörper (2) befestigten Haptik (4), die einen konkav in bezug auf die Linsenkörperkante verlaufenden Hinterkammer-Abschnitt (17) aufweist, der ein sich im Hinterkammer-Winkel (5) elastisch abzustützendes, d.h. einwärts gebogenes Abstütz-Segment (20) besitzt, einen aus der Linsenkörper-Ebene herausgebogenen und durch eine Iridektomie (12) hindurchzuführenden Durchtritts-Abschnitt (18) und einen oberhalb des Durchtritts-Abschnitts (18) parallel zur Linsenkörper-Ebene verlaufenden Vorderkammer-Abschnitt (19) aufweist,
**dadurch gekennzeichnet,**
daß die mindestens eine Haptik (4), vom Linsenkörper (2) ausgehend in Aufeinanderfolge, jeweils einen in Form einer Spirale aus dem Linsenkörper heraustretenden Hinterkammer-Abschnitt (17) mit einem Hinterkammer-Abstütz-Segment (20), einen Durchtritts-Abschnitt (18) und einen Vorderkammer-Abschnitt (19) aufweist.

2. Intraokulare Kunstlinse nach Anspruch (1),
dadurch gekennzeichnet, daß der Durchtritts-Abschnitt (18) im wesentlichen senkrecht zur Linsenebene verläuft.

3. Intraokulare Kunstlinse nach Anspruch (1),
dadurch gekennzeichnet, daß der Durchtritts-Abschnitt (18) in einem Winkel zur Linsenebene verläuft.

4. Intraokulare Kunstlinse nach Anspruch (3),
dadurch gekennzeichnet, daß der Durchtritts-Abschnitt (18) in einer im wesentlichen senkrechten Durchmesserebene zum Linsenkörper (2) verläuft.

5. Intraokulare Kunstlinse nach Anspruch 4,
dadurch gekennzeichnet, daß zwischen dem Durchtritts-Abschnitt (18) und dem Abstütz-Segment (20) des Hinterkammer-Abschnitts (17) ein dem Linsenkörper sich annähernder Einwärtsbogen (31) als Radial-Übergang vorgesehen ist.

6. Intraokulare Kunstlinse nach Anspruch 3,
dadurch gekennzeichnet, daß der Durchtritts-Abschnitt (18) in einer zum Linsenkörper (2) im wesentlichen tangentialen Ebene verläuft.

7. Intraokulare Kunstlinse nach Anspruch 1,
dadurch gekennzeichnet, daß zwei oder mehr Haptiken (4) an einem Linsenkörper (2) angeordnet sind, die im wesentlichen identisch ausgebildet sind.

8. Intraokulare Kunstlinse nach Anspruch 1,
dadurch gekennzeichnet, daß die Haptik (4) zur Abstützung am peripheren Rand der Iris (9) über deren Vorderkammer-Abschnitt (19) ausgelegt ist.

9. Instraokulare Kunstlinse nach Anspruch 8,
dadurch gekennzeichnet, daß die Haptik (4) zur Abstützung über den Vorderkammer-Abschnitt (19) in Höhe des Durchtritts-Abschnittes ausgelegt ist.

10. Intraokulare Kunstlinse nach Anspruch 1,
dadurch gekennzeichnet, daß die Haptik (4) zur Abstützung über den Vorderkammer-Abschnitt (19) im Vorderkammer-Winkel (14) ausgelegt ist.

11. Intraokulare Kunstlinse nach Anspruch 1,
dadurch gekennzeichnet, daß die Haptik (4) spiralförmig ausgebildet ist, wobei die Abstütz-Segmente (20, 25, 26) der Haptik (4) jeweils auf nur relativ kurzem Abschnitt kontaktierend ausgebildet sind.

12. Intraokulare Kunstlinse nach Anspruch 1,
dadurch gekennzeichnet, daß der Vorderkammer-Abschnitt (19) der Haptik (4) in Form einer Schlaufe ausgebildet ist und einen Anfangsarm (21) und einen gegenläufigen Abstütz-Arm (23) aufweist.

13. Intraokulare Kunstlinse nach Anspruch 12,
dadurch gekennzeichnet, daß der gegenläufige Abstütz-Arm (23) an seinem Anfangs- und Endabschnitt so geschwungen ist, daß im wesentlichen eine Zweipunkt-Anlage und -Fixierung vorhanden ist.

14. Intraokulare Kunstlinse nach Anspruch 12,
dadurch gekennzeichnet, daß von dem Linsenkörper (2) aus gesehen, der Abstütz-Arm (23) des Vorderkammer-Abschnitts (19) außerhalb des Durchtritts-Abschnitts (18), d.h. nach außen geschwungen, verläuft.

15. Intraokulare Kunstlinse nach Anspruch 12,
dadurch gekennzeichnet, daß von dem Linsenkörper (2) aus gesehen, der Abstütz-Arm (23) des Vorderkammer-Abschnitts (19) innerhalb des Durchtritts-Abschnitts (18), d.h. nach innen geschwungen, verläuft.

16. Intraokulare Kunstlinse nach einem der Ansprüche 12 bis 15, dadurch gekennzeichnet, daß der erste Teil (21) des Vorderkammer-Abschnitts (19) im wesentlichen in Spiralrichtung verläuft, wonach sich der Abstütz-Arm (23) in Spiral-Gegenrichtung anschließt.

17. Intraokulare Kunstlinse nach einem der Ansprüche 12 bis 15, dadurch gekennzeichnet, daS der erste Teil (27) des Vorderkammer-Abschnitts (19) im wesentlichen in der Spiral-Gegenrichtung verläuft, wonach sich der Abstütz-Arm (23) im wesentlichen in Spiralrichtung anschließt.

18. Intraokulare Kunstlinse nach Anspruch 13,
dadurch gekennzeichnet, daß das Abstütz-Ende (24) des Vorderkammer-Abschnitts (19) eine insbesondere nach innen sich erstreckende Erweiterung aufweist und eine Handhabungsöffnung (30) besitzt.

19. Intraokulare Kunstlinse nach Anspruch 13,
dadurch gekennzeichnet, daß das Abstütz-Ende (24) in Form eines im wesentlichen kreisförmigen Bogens ausgebildet ist.

20. Intraokulare Kunstlinse nach Anspruch 1,
dadurch gekennzeichnet, daß der Vorderkammer-Abschnitt (19) einen im wesentlichen im Spiralverlauf geführten Abstütz-Arm (28) und einen gegenläufigen, im wesentlichen in gleicher Ebene liegenden Haken-Arm (29) aufweist.

21. Intraokulare Kunstlinse nach einem der Ansprüche 1 oder 20, dadurch gekennzeichnet, daß der Vorderkammer-Abschnitt (19) aus einem im wesentlichen in Spiralverlauf geführten ersten Abstütz-Arm (28) und einem im Verhältnis zum Durchtritts-Abschnitt (18) spiegelbildlich ausgebildeten zweiten Abstütz-Arm (28) besteht, wobei die beiden Arme (28) zum Hindurchführen durch die Iridektomie (12) in eine Ebene mit dem Durchtritts-Abschnitt (18) zusammenlegbar sind.

22. Intraokulare Kunstlinse nach Anspruch 1,
dadurch gekennzeichnet, daß der Vorderkammer-Abschnitt (19) mindestens einen oberhalb des Durchtritts-Abschnitts im wesentlichen tangential zu diesem geführten Einhängearm (32) aufweist.

23. Interaokulare Kunstlinse nach Anspruch 22,
dadurch gekennzeichnet, daß der mindestens eine Einhängearm (32) gegenläufig zum Hinterkammer-Abschnitt (17) geführt ist.

24. Interaokulare Kunstlinse nach Anspruch 22,
dadurch gekennzeichnet, daß der Einhängearm (32) gleichläufig zum Hinterkammer-Abschnitt (17) geführt ist.

25. Interaokulare Kunstlinse nach Anspruch 22,
dadurch gekennzeichnet, daß der Einhängearm (32) oberhalb des Durchtritts-Abschnitts mindestens einen im wesentlichen radial weisenden Querarm (33) aufweist.

26. Interaokulare Kunstlinse nach Anspruch 25,
dadurch gekennzeichnet, daß zwei im wesentlichen identische, radial gegenläufige Querarme (33) vorgesehen sind.

27. Intraokulare Kunstlinse nach Anspruch 22,
dadurch gekennzeichnet, daß zwei tangential gegenläufige Einhängearme vorgesehen sind, die eine im wesentlichen zum Durchtritts-Abschnitt symmetrische Aufhängung (34) bilden.

28. Intraokulare Kunstlinse nach Anspruch 1,
dadurch gekennzeichnet, daß der Vorderkammer-Abschnitt (19) als Anfangsabschnitt einen Radialteil (36) aufweist, der im wesentlichen radial und gleichzeitig parallel zur Linse oberhalb des Durchtritts-Abschnitts verläuft.

29. Intraokulare Kunstlinse nach Anspruch 28,
dadurch gekennzeichnet, daß der Radialteil im Verhältnis zur Linse (2) radial nach außen oder radial nach innen verläuft:

30. Intraokulare Kunstlinse nach Anspruch 1,
dadurch gekennzeichnet, daß das Hinterkammer-Abstütz-Segment (20) des Hinterkammer-Abschnitts (17) im wesentlichen in der gleichen Ebene mit dem Linsenkörper (2) liegt, daß an sein von der Peripherie nach innen geschweiftes Ende sich der im wesentlichen senkrecht verlaufendende Durchtritts-Abschnitt (18) anschließt, und an diesen wiederum der Vorderkammer-Abschnitt (19).

31. Intraokulare Kunstlinse nach Anspruch 30,
dadurch gekennzeichnet, daß der Hinterkammer-Abschnitt (17) der Haptik (4) von seinem Ansatzpunkt am Linsenkörper (2) bis zum Durchtritts-Abschnitt (18), in horizontalem Augen-Achsen-Schnitt gesehen, in einer ca. 10°-Neigung zur Optik-Ebene verläuft.

32. Intraokulare Kunstlinse nach Anspruch 1,
dadurch gekennzeichnet, daß der Linsenkörper (2) mit der mindestens einen Haptik (4) einteilig ausgebildet ist.

33. Intraokulare Kunstlinse nach Anspruch 1,
dadurch gekennzeichnet, daß der Linsenkörper (2) mit der mindestens einen Haptik (4) mehrstückig ausgebildet ist, wobei die mindestens eine Haptik (4) am Linsenkörper (2) in an sich bekannter Weise angeklebt ist.

34. Intraokulare Kunstlinse nach Anspruch 1,
dadurch gekennzeichnet, daß nur eine Haptik (4) an einem Linsenkörper (2) vorgesehen ist, wobei die Abstütz-Segmente (20, 25, 26) so ausgebildet sind, daß sie ungefähr im gleichen Umfangssektor klemmend wirksam sind.

35. Verfahren zum Herstellen der Intraokularen Kunstlinse nach den Ansprüchen 1 bis 34,
**dadurch gekennzeichnet,**
- daß die Haptiken (4) mit allen drei Abschnitten (17, 18, 19) komplett in einer Ebene liegend herausgearbeitet werden,
- daß danach durch Verschwenk-Verformen zumindest des Durchtritt-Abschnitts (18) die Funktionslagen der Haptik-Abschnitte hergestellt werden.

36. Verfahren nach Anspruch 35,
dadurch gekennzeichnet, daß die Herausarbeitung spanabhebend erfolgt.

37. Verfahren nach Anspruch 35,
dadurch gekennzeichnet, daß das Biegen in die Funktionslage der Haptik-Abschnitte (18, 19) durch thermische Verformung erfolgt.

38. Verfahren nach Anspruch 35,
dadurch gekennzeichnet, daß bei einstückiger Herstellung der Kunstlinse, die Haptiken (4) mit allen ihren Abschnitten in der Linsenkörper-Ebene liegen und anschließend aus dieser heraus verformt werden.

## Claims

1. Intraocular artificial lens for replacement of the lens of the eye, with a lens body (2) to be disposed in the posterior chamber of the eye, and with at least one haptic fastener (4) which is fixed to the lens body (2) and exhibits a posterior chamber portion (17) running concavely in relation to the edge of the lens body, which posterior chamber portion has a supporting segment (20) which is to be supported elastically in the posterior chamber angle (5) - i.e. is inwardly curved, and exhibits a penetration portion (18) curved out of the plane of the lens body and to be passed through an iridectomy (12), and an anterior chamber portion (19) running above the penetration portion (18) parallel with the plane of the lens body, characterised in that starting from the lens body (2) in succession, the at least one haptic fastener (4) exhibits in each case a posterior chamber portion (17) emerging from the lens body in the form of a spiral with a posterior chamber supporting segment (20), a penetration portion (18) and an anterior chamber portion (19).

2. Intraocular artificial lens according to claim 1, characterised in that the penetration portion (18) runs essentially perpendicular to the plane of the lens.

3. Intraocular artificial lens according to claim 1, characterised in that the penetration portion (18) runs at an angle to the plane of the lens.

4. Intraocular artificial lens according to claim 3, characterised in that the penetration portion (18) runs in an essentially perpendicular diametral plane to the lens body (2).

5. Intraocular artificial lens according to claim 4, characterised in that an inward arc (31) approaching the lens body is provided as radial transition between the penetration portion (18) and the supporting segment (20) of the posterior chamber portion (17).

6. Intraocular artificial lens according to claim 3, characterised in that the penetration portion (18) runs in a plane essentially tangential to the lens body (2).

7. Intraocular artificial lens according to claim 1, characterised in that two or more haptic fasteners (4), which are essentially identical, are disposed on a lens body (2).

8. Intraocular artificial lens according to claim 1, characterised in that the haptic fastener (4) is designed for support on the peripheral rim of the iris (9) by means of its anterior chamber portion (19).

9. Intraocular artificial lens according to claim 8, characterised in that the haptic fastener (4) is designed for support by means of the anterior chamber portion (19) level with the penetration portion.

10. Intraocular artificial lens according to claim 1, characterised in that the haptic fastener (4) is designed for support by means of the anterior chamber portion (19) in the anterior chamber angle (14).

11. Intraocular artificial lens according to claim 1, characterised in that the haptic fastener (4) is spiral-shaped, and the supporting segments (20, 25, 26) of the haptic fastener (4) are each embodied so that they only make contact over a relatively short portion.

12. Intraocular artificial lens according to claim 1, characterised in that the anterior chamber portion (19) of the haptic fastener (4) takes the form of a loop and exhibits a starting arm (21) and a supporting arm (23) running in the opposite direction.

13. Intraocular artificial lens according to claim 12, characterised in that at its starting and end portion the supporting arm (23) running in the opposite direction is pivoted so that two-point contact and fixture is essentially achieved.

14. Intraocular artificial lens according to claim 12, characterised in that, viewed from the lens body (2), the supporting arm (23) of the anterior chamber portion (19) runs outside the penetration portion (18), i.e. is pivoted outwards.

15. Intraocular artificial lens according to claim 12, characterised in that, viewed from the lens body (2), the supporting arm (23) of the anterior chamber portion (19) runs inside the penetration portion (18), i.e. is pivoted inwards.

16. Intraocular artificial lens according to one of claims 12 to 15, characterised in that the first part (21) of the anterior chamber portion (19) essentially runs in a spiral direction, after which the supporting arm (23) adjoins in the opposite spiral direction.

17. Intraocular artificial lens according to one of claims 12 to 15, characterised in that the first part (27) of the anterior chamber portion (19) essentially runs in the opposite spiral direction, after which the supporting arm (23) adjoins essentially in the spiral direction.

18. Intraocular artificial lens according to claim 13, characterised in that the supporting end (24) of the anterior chamber portion (19) exhibits a widening extending in particular inwards, and has a manipulating opening (30).

19. Intraocular artificial lens according to claim 13, characterised in that the supporting end (24) takes the form of an essentially circular arc.

20. Intraocular artificial lens according to claim 1, characterised in that the anterior chamber portion (19) exhibits a supporting arm (28) essentially guided in the spiral, and a hook arm (29) running in the opposite direction and essentially lying in the same plane.

21. Intraocular artificial lens according to one of claims 1 or 20, characterised in that the anterior chamber portion (19) consists of a first supporting arm (28) essentially guided in a spiral, and of a second supporting arm (28) embodied as a mirror image in relation to the penetration portion (18), and the two arms (28) can be swung into a plane with the penetration portion (18) for passage through the iridectomy (12).

22. Intraocular artificial lens according to claim 1, characterised in that the anterior chamber portion (19) exhibits at least one attaching arm (32) guided above the penetration portion essentially tangentially to the latter.

23. Intraocular artificial lens according to claim 22, characterised in that the at least one attaching arm (32) is guided running in the opposite direction to the posterior chamber portion (17).

24. Intraocular artificial lens according to claim 22, characterised in that the attaching arm (32) is guided running in the same direction to the posterior chamber portion (17).

25. Intraocular artificial lens according to claim 22, characterised in that above the penetration portion the attaching arm (32) exhibits at least one essentially radially directed transverse arm (33).

26. Intraocular artificial lens according to claim 25, characterised in that two essentially identical transverse arms (33) are provided which run in opposite directions radially.

27. Intraocular artificial lens according to claim 22, characterised in that two attaching arms are provided which run in opposite directions tangentially and form a suspension (34) essentially symmetrical to the penetration portion.

28. Intraocular artificial lens according to claim 1, characterised in that as starting portion the anterior chamber portion (19) exhibits a radial part (36) which runs essentially radially and at the same time parallel to the lens above the penetration portion.

29. Intraocular artificial lens according to claim 28, characterised in that the radial part runs radially outwards or radially inwards in relation to the lens (2).

30. Intraocular artificial lens according to claim 1, characterised in that the posterior chamber supporting segment (20) of the posterior chamber portion (17) lies essentially in the same plane with the lens body (2), in that at its end swept inwards from the periphery it is adjoined by the penetration portion (18) running essentially perpendicularly, and this in turn is adjoined by the anterior chamber portion (19).

31. Intraocular artificial lens according to claim 30, characterised in that, viewed in the horizontal eye axis section, from its point of attachment on the lens body (2) to the penetration portion (18) the posterior chamber portion (17) of the haptic fastener (4) runs with an inclination of approximately 10° to the optical plane.

32. Intraocular artificial lens according to claim 1, characterised in that the lens body (2) is formed in one piece with the at least one haptic fastener (4).

33. Intraocular artificial lens according to claim 1, characterised in that the lens body (2) is formed in more than one piece with the at least one haptic fastener (4), and the at least one haptic fastener (4) is bonded to the lens body (2) in a manner known per se.

34. Intraocular artificial lens according to claim 1, characterised in that only one haptic fastener (4) is provided on a lens body (2), and the supporting segments (20, 25, 26) are embodied so that they have a clamping effect roughly in the same circumferential sector.

35. Method for production of the intraocular artificial lens according to claims 1 to 34, characterised in that the haptic fasteners (4) with all three portions (17, 18, 19) are fabricated complete lying in one plane, in that afterwards the functional positions of the haptic portions are produced by rotational forming of at least the penetration portion (18).

36. Method according to claim 35, characterised in that the fabrication is effected by removing material.

37. Method according to claim 35, characterised in that the bending into the functional position of the haptic portions (18, 19) is effected by thermal forming.

38. Method according to claim 35, characterised in that when the artificial lens is produced in one piece, the haptic fasteners (4) with all their portions lie in the plane of the lens body and are then formed out of this plane.

## Revendications

1. Lentille artificielle intraoculaire, destinée à remplacer la lentille de l'oeil, comportant un corps de lentille (2), destinée à être disposée dans la chambre postérieure de l'oeil, et au moins un étrier de fixation 4, assujetti au corps de lentille (2), et qui comporte une segment de chambre postérieure (17), s'étendant d'une manière concave par rapport à l'arête du corps de lentille, ce segment de chambre postérieure ayant un segment d'appui (20), destinée à assurer un appui élastique dans le sillon de chambre antérieure (5), c'est-à-dire tournant sa convexité vers l'intérieur, et qui comporte un segment de passage (18), repliée vers l'extérieur à partir du plan du corps de la lentille et destiné à traverser une iridectomie (12), ainsi qu'un segment de chambre antérieure (19), s'étendant, au-dessus du segment de passage (18), parallèlement au plan du corps de la lentille, caractérisée en ce que l'au moins un étrier de fixation (4) comporte chacun, en partant du corps de la lentille (2), et successivement, un segment de chambre postérieure (17), qui sort du corps de la lentille sous forme d'une spirale et qui comporte un segment d'appui de chambre postérieure (20), ainsi qu'un segment de passage (18) et un segment de chambre antérieure (19).

2. Lentille artificielle intraoculaire selon la revendication 1, caractérisée en ce que le segment de passage (18) s'étend d'une manière essentiellement perpendiculaire au plan de la lentille.

3. Lentille artificielle intraoculaire selon la revendication 1, caractérisée en ce que le segment de passage (18) s'étend en faisant un angle avec le plan de la lentille.

4. Lentille artificielle intraoculaire selon la revendication 3, caractérisée en ce que le segment de passage (18) s'étend dans un plan diamétral essentiellement perpendiculaire au corps de la lentille (2).

5. Lentille artificielle intraoculaire selon la revendication 4, caractérisée en ce qu'on prévoit, entre le segment de passage (18) et le segment d'appui (20) du segment de chambre postérieure (17), un arceau (31) tournant sa convexité vers l'intérieur, qui se rapproche du corps de la lentille et qui sert de transition radiale.

6. Lentille artificielle intraoculaire selon la revendication 3, caractérisée en ce que le segment de passage (18) s'étend dans un plan essentiellement tangentiel au corps de lentille (2).

7. Lentille artificielle intraoculaire selon la revendication 1, caractérisée en ce que sur un corps de lentille (2) sont disposés au moins deux étriers de fixation (4), qui pour l'essentiel ont une configuration identique.

8. Lentille artificielle intraoculaire selon la revendication 1, caractérisée en ce que l'étrier de fixation 4 est conçu de façon à assurer un appui contre le bord périphérique de l'iris (9) par l'intermédiaire de son segment de chambre antérieure (19).

9. Lentille artificielle intraoculaire selon la revendication 8, caractérisée en ce que l'étrier de fixa-tion (4) est conçu pour assurer un appui par l'intermédiaire du segment de chambre antérieure (19) à la hauteur du segment de passage.

10. Lentille artificielle intraoculaire selon la revendication 1, caractérisée en ce que l'étrier de fixation est conçu pour assurer un appui par l'intermédiaire du segment de chambre antérieure (19) dans le sillon de chambre antérieure (14).

11. Lentille artificielle intraoculaire selon la revendication 1, caractérisée en ce que l'étrier de fixa-tion (4) est conçu en spirale, les segments d'appui (20, 25, 26) de l'étrier de fixation (4) étant chacun conçu de façon à n'entrer en contact que sur un segment relativement court.

12. Lentille artificielle intraoculaire selon la revendication 1, caractérisée en ce que le segment de chambre antérieure (19) de l'étrier de fixation 4 est conçu sous forme d'une boucle, et comporte un bras de départ (21) et un bras d'appui (23) en sens contraire.

13. Lentille artificielle intraoculaire selon la revendication 12, caractérisée en ce que le bras d'appui en sens contraire (23) est arqué en son segment de départ et en son segment d'extrémité de façon à avoir essentiellement un soutien et une immobilisation en deux points.

14. Lentille artificielle intraoculaire selon la revendication 12, caractérisée en ce que, quand on regarde à partir du corps de lentille (2), le bras d'appui (23) du segment de chambre antérieure (19) s'étend à l'extérieur du segment de passage (18), c'est-à-dire est arqué vers l'extérieur.

15. Lentille artificielle intraoculaire selon la revendication 12, caractérisée en ce que, quand on regarde à partir du corps de lentille (2), le bras d'appui (23) du segment de chambre antérieure (19) s'étend à l'intérieur du segment de passage 18, c'est-à-dire est arqué vers l'intérieur.

16. Lentille artificielle intraoculaire selon l'une des revendications 12 à 15, caractérisée en ce que la première partie (21) du segment de chambre antérieure (19) s'étend essentiellement en spirale, en étant suivi du bras d'appui (23), qui s'étend en spirale dans le sens inverse.

17. Lentille artificielle intraoculaire selon l'une des revendications 12 à 15, caractérisée en ce que la première partie du segment de chambre antérieure s'étend essentiellement dans le sens inverse en spirale, le bras d'appui (23) suivant, essentiellement dans le sens de la spirale.

18. Lentille artificielle intraoculaire selon la revendication 13, caractérisée en ce que l'extrémité d'appui (24) du segment de chambre antérieure (19) possède un élargissement, s'étendant en particulier vers l'intérieur, et comporte une ouverture de manipulation (30).

19. Lentille artificielle intraoculaire selon la revendication 13, caractérisée en ce que l'extrémité d'appui (24) est configurée sous forme d'un arceau essentiellement circulaire.

20. Lentille artificielle intraoculaire selon la revendication 1, caractérisée en ce que le segment de chambre antérieure (19) comporte un bras d'appui (28), dont le tracé est essentiellement en spirale, et un bras d'accrochage (29), en sens contraire, disposé essentiellement dans le même plan.

21. Lentille artificielle intraoculaire selon l'une des revendications 1 à 20, caractérisée en ce que le seg-ment de chambre antérieure (19) est constitué d'un premier bras d'appui (28), dont le tracé est essentiellement en spirale, et d'un deuxième bras d'appui (28), formé d'une manière symétrique comme en un miroir par rapport au segment de passage (18), les deux bras (28) pouvant, pour permettre une traversée de l'iridectomie, être réunis dans un plan avec le segment de passage (18).

22. Lentille artificielle intraoculaire selon la revendication 1, caractérisée en ce que le segment de chambre antérieure (19) comporte au moins un bras de suspension (32), passe, au-dessus du segment de passage, d'une manière essentiellement tangentielle à ce dernier.

23. Lentille artificielle intraoculaire selon la revendication 22, caractérisée en ce que l'au moins un bras de suspension (32) passe en sens contraire du segment de chambre postérieure (17).

24. Lentille artificielle intraoculaire selon la revendication 22, caractérisée en ce que le bras de suspension (32) est dans le même sens que le segment de chambre postérieure (17).

25. Lentille artificielle intraoculaire selon la revendication 22, caractérisée en ce que le bras de suspension (32) présente, au-dessus du segment de passage, au moins un bras transversal (33), tourné essentiellement d'une manière radiale.

26. Lentille artificielle intraoculaire selon la revendication 25, caractérisée en ce qu'on prévoit deux bras transversaux (33), et essentiellement identiques, radialement opposés.

27. Lentille artificielle intraoculaire selon la revendication 22, caractérisée en ce qu'on prévoit deux bras de suspension opposés dans le sens tangentiel, qui forment une suspension (34), essentiellement symétrique par rapport au segment de passage.

28. Lentille artificielle intraoculaire selon la revendication 1, caractérisée en ce que le segment de chambre antérieure (19) comporte en tant que segment de départ une partie radiale (36), qui s'étend au-dessus du segment de passage, d'une manière essentiellement radiale et simultanément parallèle à la lentille.

29. Lentille artificielle intraoculaire selon la revendication 28, caractérisée en ce que la partie radiale s'étend, par rapport à la lentille (2), radialement vers l'extérieur ou radialement vers l'intérieur.

30. Lentille artificielle intraoculaire selon la revendication 1, caractérisée en ce que le segment d'appui de chambre postérieure (20) du segment de chambre postérieure (17) se trouve essentiellement dans le même plan que le corps de lentille (2), son extrémité incurvée vers l'intérieur à partir de la périphérie se poursuivant par le segment de passage (18), qui s'étend d'une manière essentiellement perpendiculaire, ce dernier étant, pour sa part, suivi du segment de chambre antérieure (19).

31. Lentille artificielle intraoculaire selon la revendication 30, caractérisée en ce que le segment de chambre postérieure (17) de l'étrier de fixation s'étend, depuis son point d'appui sur le corps de lentille (2) jusqu'au segment de passage (18), et quand on regarde en coupe horizontale selon l'axe de l'oeil, avec une inclinaison d'environ 10° par rapport au plan de l'optique.

32. Lentille artificielle intraoculaire selon la revendication 1, caractérisée en ce que le corps de lentille (2) est réalisé d'une manière monobloc avec au moins un étrier de fixation (4).

33. Lentille artificielle intraoculaire selon la revendication 1, caractérisée en ce que le corps de lentille (2) est conçu en plusieurs morceaux avec au moins un étrier de fixation (4), l'au moins un étrier de fixation 4 étant collé, d'une manière connue en soi, au corps de lentille (2).

34. Lentille artificielle intraoculaire selon la revendication 1, caractérisée en ce qu'on ne prévoit qu'un étrier de fixation (4) sur un corps de lentille (2), les segments d'appui (20, 25, 26) étant réalisés de façon à avoir un effet de serrage approximativement dans le même secteur périphérique.

35. Procédé pour fabriquer la lentille artificielle intraoculaire selon les revendications 1 à 34, caractérisé en ce que les étriers de fixation (4), avec les trois segments (17, 18, 19), sont entièrement façonnés dans un plan, puis l'on obtient les positions fonctionnelles des segments de l'étrier de fixation, par façonnage par basculement au moins du segment de passage (18).

36. Procédé selon la revendication 35, caractérisé en ce que la fabrication s'effectue avec enlèvement de copeaux.

37. Procédé selon la revendication 35, caractérisé en ce que l'incurvation dans la position fonctionnelle des segments de l'étrier de fixation (18, 19) s'effectue par déformation thermique.

38. Procédé selon la revendication 35, caractérisé en ce que, lors de la fabrication monobloc de la lentille artificielle, les étriers de fixation (4) se trouvent, avec tous leurs segments, dans le plan du corps de la lentille, puis sont façonnés à partir de ce dernier.
